# EUROPÄISCHE PATENTSCHRIFT

(11) **EP 3 313 472 B1**
(45) Veröffentlichungstag und Bekanntmachung des Hinweises auf die Patenterteilung: **02.10.2019**
(21) Anmeldenummer: 16721081.4
(22) Anmeldetag: 09.05.2016
(51) Int. Cl.: A61M 1/16, A45F 3/14

(54) **TRAGEVORRICHTUNG FÜR EINE GASAUSTAUSCHVORRICHTUNG**
CARRYING DEVICE FOR A GAS EXCHANGE DEVICE
DISPOSITIF DE SUPPORT POUR UN DISPOSITIF D'ÉCHANGE GAZEUX

(30) Priorität: 29.06.2015 EP 15001921
(43) Veröffentlichungstag der Anmeldung: 02.05.2018
(60) Teilanmeldung: 19187389.2
(73) Patentinhaber: Novalung GmbH, 74076 Heilbronn (DE)
(72) Erfinder: MATHEIS, Georg, 74076 Heilbronn (DE); NOVOSEL, Esther, 70197 Stuttgart (DE); BEUTER, Reinhold, 72414 Rangendingen (DE); SCHNEIDER, Jörg, 82319 Starnberg (DE); BOGENSCHÜTZ, Josef, 72406 Bisingen (DE)
(74) Vertreter: Graf von Stosch, Andreas
(86) Internationale Anmeldenummer: PCT/EP2016/000751
(87) Internationale Veröffentlichungsnummer: WO 2017/001034

(56) Entgegenhaltungen:
- WO-A1-2015/100288
- CA-A1- 2 797 702
- DE-A1-102009 050 406
- US-A- 5 250 027
- US-A1- 2008 014 115
- US-A1- 2012 138 058
- US-A1- 2012 209 228
- US-A1- 2014 276 498
- US-B2- 7 718 144

## Beschreibung

### Hintergrund der Erfindung

Die Erfindung betrifft eine Tragevorrichtung sowie ein Tragesystem.

Es sind Gasaustauschvorrichtungen bekannt, bei denen es sich um Begasungs- bzw. Entgasungsvorrichtungen handelt, bei denen ein oder mehrere Gase von einem Medium in ein anderes Medium übertreten können, bzw. um Vorrichtungen, die den Austausch eines oder mehrerer Gase zwischen zwei Medien ermöglichen. Solche Vorrichtungen finden Anwendung in der Chemie, der Biotechnologie und der Medizin. Ein wichtiger Einsatzzweck in der Medizin ist die Anreicherung einer biologischen Flüssigkeit, insbesondere von Blut, mit Sauerstoff und/oder die Entfernung, also Abreicherung, von Kohlenstoffdioxid aus der Flüssigkeit, speziell Blut. Solche Maßnahmen werden bspw. bei der Behandlung von verschiedenen Lungenerkrankungen notwendig. Solche Maßnahmen können weiterhin z.B. auch bei akutem Lungenversagen, sowie zum Ersatz der Lunge, während deren Umgehung mit einem extrakorporalen Kreislauf, mit unterschiedlichem Ausmaß bei der mechanischen Herzunterstützung und zur Ermöglichung am stillgestellten Herzen zu operieren, notwendig sein.

Derzeit ist es die einzige langfristig effektive Therapieoption für Patienten mit endgradiger funktioneller Lungenerkrankung, eine Lungentransplantation vorzunehmen. Eine andere medizinische Lösung, um dauerhaft die Funktion der Lunge zu ersetzen, existiert hingegen nicht. Bei Patienten, die unter chronischen Lungenerkrankungen, wie COPD bzw. AECOPD, leiden und nicht oder nicht unmittelbar für eine Lungentransplantation in Betracht kommen, besteht daher ein Bedürfnis nach künstlichen Lungenunterstützungs- oder Lungenersatzverfahren.

Um ein derartiges Lungenunterstützungs- bzw. Lungenersatzverfahren zu ermöglichen, sind so genannte Blutgastauscher aus dem Stand der Technik bekannt.

Ein Blutgastauscher, auch als Oxygenator oder künstliche Lunge bezeichnet, wird entweder zur vollständigen, kurzzeitigen Übernahme der Lungenfunktion während einer Operation am offenen Herzen oder als vollständige oder teilweise, langfristige Unterstützung der Lunge auf der Intensivstation eingesetzt. Die Hauptfunktion eines Blutgastauschers besteht in der Abgabe von Sauerstoff an das Blut (Oxygenierung) und in der Aufnahme von Kohlenstoffdioxid aus dem Blut (Decarboxylierung).

Neben einer Anwendung zur Oxygenierung und Decarboxylierung ist es in einigen Therapieansätzen bereits ausreichend, lediglich eine Decarboxylierung vorzunehmen. Dabei handelt es sich um eine so genannte extrakorporale Reduktion von CO₂ (ECCO₂R). Dazu wird über einen venösen Zugang des Patienten kontinuierlich Blut entnommen, extrakorporal durch einen Blutgastauscher gepumpt, dort von CO₂ gereinigt und dem Patienten venös wieder zugeführt.

Für den Gasaustausch in derartigen Blutgastauschern wird üblicherweise Sauerstoff verwendet, der in jedem Krankenhaus entweder direkt über einen Wandanschluss oder Sauerstoffflaschen zur Verfügung steht. Der Sauerstoff wird dann über einen regelbaren Gasblender dem Blutgastauscher zugeführt.

Pumpsysteme für ECCO₂R - Anwendungen werden grundsätzlich am stationären Stromnetz betrieben. Im Falle eines Notfalls, wie beispielsweise einem Stromausfall, kann eine Notstromversorgung mittels Batteriebetrieb aufrechterhalten werden. Da Krankeneinrichtungen in der Regel über Notstromgeneratoren verfügen, ist ein Batteriebetrieb zeitlich jedoch limitiert und bei den bekannten Gasaustauschvorrichtungen in der Regel nicht zum Dauerbetrieb oder zum mobilen Betrieb ausgelegt. Ein bekanntes Blutgastauschsystem, das zwar über eine mobile Stromversorgung verfügt, erfordert jedoch das Mitführen einer rollbaren Trägereinrichtung, um die Vielzahl verschiedener, zum Teil sperriger, Komponenten des Blutgastauschers und, insbesondere, Gasflaschen mitzuführen.

Auf Grund ihrer Größe, ihres Gewichts und auch wegen der Anordnung von Anschlüssen beispielsweise für blutführende Schläuche, beschränkte sich bislang der Einsatz der bekannten Gasaustauschvorrichtungen daher im Wesentlichen auf stationäre Anwendungen, bei denen der Patient - wach oder sediert - im Bett liegt. Patienten mit chronischen Lungenerkrankungen, die auf eine dieser bekannten Gasaustauschvorrichtungen angewiesen sind, sind somit stark in ihrer Mobilität eingeschränkt. Dies vermindert nicht nur die Lebensqualität der Patienten erheblich. Auch moderne Therapieansätze, die eine Mobilisierung der Patienten anstreben, sind nicht durchführbar.

Gasaustauschvorrichtungen, die grundsätzlich für einen mobilen Einsatz geeignet sind, sind Gegenstand neuester Entwicklungen. Um für einen Patienten, der auf eine derartige Gasaustauschvorrichtung angewiesen ist, Mobilität zu gewähren, ist es wünschenswert, dass ein sicherer Transport der Gasaustauschvorrichtung erfolgen kann. Bei der Verwendung üblicher Trägereinrichtungen, beispielsweise ähnlich eines rollbaren Infusionsständers, besteht dabei jedoch grundsätzlich die Gefahr, dass die Trägereinrichtung unvorhersehbar von dem Patienten getrennt wird, beispielsweise wenn der Patient stürzt oder Personen oder Objekte zwischen den Patienten und das Tragesystem geraten. Im schlimmsten Falle kann dies dazu führen, dass ein extrakorporaler Blutkreislauf unterbrochen oder gar zerstört wird. Dies kann schwere gesundheitliche Konsequenzen für den Patienten mit sich bringen, bis hin zu Blutverlust, einer Sauerstoffunterversorgung oder einer Kohlenstoffdioxid-Vergiftung mit schwerwiegenden Folgen.

Bekannte Gasaustauschvorrichtungen zur Unterstützung der Lungenfunktion weisen dabei grundsätzlich dieselben Nachteile auf, wie sie auch aus Lungenersatzvorrichtungen bekannt sind. Auch hierbei muss eine Gasflasche transportiert werden und verschiedene Gerätschaften müssen am Körper verteilt getragen oder separat mittransportiert werden.

Relevanter Stand der Technik ist in den folgenden Dokumenten offenbart: DE102009050406 A1, US2008/014115 A1, US7718144 B2 und WO2015/100288 A1.

### Kurze Beschreibung der Erfindung

Der vorliegenden Erfindung liegt die Aufgabe zugrunde, zumindest einen der Nachteile des Standes der Technik zu vermeiden und einen sicheren Transport einer transportablen Gasaustauscheinrichtung zu ermöglichen. Es ist zudem eine Aufgabe, zu ermöglichen, dass ein Patient eine Gasaustauschvorrichtung auch über einen längeren Zeitraum hinweg, vorzugsweise direkt, an dem Patientenkörper transportieren kann. Insbesondere soll ein Transport einer Gasaustauschvorrichtung ermöglicht werden, die für den mobilen, tragbaren Einsatz an einem Patientenkörper konzipiert ist, beispielsweise durch eine besondere kompakte Ausgestaltung, zur Verwendung als Lungenunterstützungssystem.

Diese Aufgabe wird durch eine Tragevorrichtung gemäß Anspruch 1 sowie ein Tragesystem gemäß Anspruch 9 gelöst. Vorteilhafte Weiterbildungen der Erfindung sind Gegenstand der abhängigen Ansprüche.

Ein erster Aspekt der vorliegenden Erfindung betrifft eine Tragevorrichtung für eine tragbare Gasaustauschvorrichtung, insbesondere eine tragbare Gasaustauschvorrichtung als Lungenunterstützungssystem in Form einer Gasversorgung. Die Tragevorrichtung weist wenigstens einen ersten Halteriemen auf, der dazu ausgelegt ist, um einen Torso eines Patienten geführt zu werden und auf diese Weise eine Abstützung der Tragevorrichtung an dem Patienten ermöglicht. Zudem weist die Tragevorrichtung wenigstens ein Befestigungsmittel auf, das zur Befestigung einer tragbaren Gasaustauschvorrichtung an der Tragevorrichtung ausgelegt ist. Eine Gasaustauschvorrichtung kann somit sicher und komfortabel von einem Patienten getragen werden. Unter die Bezeichnung "Gasaustauschvorrichtung" fallen dabei im Sinne der vorliegenden Erfindung insbesondere solche Vorrichtungen, die eine Gasversorgung mit einem oder mehreren vorbestimmten Gasen ermöglicht, beispielsweise in Form einer Zuluft-Komponente. Wird die erfindungsgemäße Vorrichtung insbesondere lediglich zur Unterstützung und nicht zum vollständigen Ersatz der Lungenfunktion verwendet, so kann die Notwendigkeit wegfallen, zusätzliche Gasflaschen, insbesondere zur Sauerstoffversorgung, mitzuführen. Dadurch kann die erfindungsgemäße Tragevorrichtung ermöglichen, eine Gasaustauschvorrichtung, vorzugsweise mit allen zur Lungenunterstützung notwendigen Komponenten, direkt an einem Patientenkörper zu tragen. Dies kann eine Mobilität der Patienten erheblich steigern. Zudem kann wenigstens ein zweiter Halteriemen vorgesehen sein, der dazu ausgelegt ist, um wenigstens eine erste Schulter eines Patienten geführt zu werden. Auf diese Weise kann eine Gewichtskraft der Tragevorrichtung und daran befestigter Komponenten abgeleitet werden.

Als "Riemen" werden im Rahmen der vorliegenden Erfindung Abschnitte der Tragevorrichtung bezeichnet, die als separate Einheiten unabhängig voneinander ausgebildet sind. Alternativ können die Riemen auch einstückig miteinander und/oder mit einer weitergehenden Tragestruktur ausgebildet sein.

Durch Vorsehen von wenigstens zwei Riemen, einen um den Torso eines Patienten führbaren Riemen und einem um wenigstens eine Schulter führbaren Riemen, kann eine sichere Befestigung der Tragevorrichtung an einem Patientenkörper ermöglicht werden. So kann bspw. der zweite Halteriemen derart ausgelegt sein, dass er diagonal über den Torso und eine erste Schulter eines Patienten geführt werden kann. Der erste Halteriemen kann derart ausgelegt sein, dass er in einer im Wesentlichen horizontalen Richtung bspw. um eine Taille eines Patienten geführt werden kann, so dass eine Befestigung der Tragevorrichtung ähnlich einem Drei-Punkte-Gurtsystem vorgenommen werden kann. Der zweite Halteriemen ist mit dem ersten Halteriemen verbunden oder wird, zumindest abschnittsweise, um den ersten Halteriemen geführt.

Eine mobile bzw. tragbare Gasaustauschvorrichtung kann an dem ersten Halteriemen mittels des Befestigungsmittels befestigt werden. Auf diese Weise kann eine tragbare Gasaustauschvorrichtung sicher von einem Patienten direkt am Patientenkörper getragen werden. Dies kann eine Mobilität des Patienten erhöhen und so eine Genesung oder Therapie eines Patienten fördern. Zudem kann eine Gefahr der Beschädigung der tragbaren Gasaustauschvorrichtung bzw. eines extrakorporalen Blutkreislaufs reduziert werden. Das Befestigungsmittel zur Befestigung der Gasaustauschvorrichtung kann dabei in einem vorderen Bereich des ersten Halteriemens ausgebildet sein. Das Befestigungsmittel kann einstückig mit einem der Halteriemen, vorzugsweise dem ersten Halteriemen, ausgebildet sein, oder als separates Element an einem der Halteriemen befestigt sein.

Gemäß einiger Ausführungsformen der vorliegenden Erfindung kann wenigstens der erste und/oder der zweite Halteriemen ein Mittel zur Anpassung, beispielsweise der Länge, des ersten und/oder zweiten Halteriemens aufweisen. Auf diese Weise kann eine Anpassung der Tragevorrichtung auf den individuellen Körperbau eines Patienten erfolgen. Dies kann einen Tragekomfort erhöhen. Eine Anpassung der Länge eines Riemens, beispielsweise des zweiten Halteriemens, kann beispielsweise dadurch ermöglicht sein, dass der Riemen zwei mit jeweils einem Verschlussmittelabschnitt versehene Schenkel oder Riemenabschnitte aufweist, wobei die Verschlussmittelabschnitte zueinander komplementär sind und eine Verbindung, insbesondere eine reversibel lösbare und schließbare Verbindung, miteinander herstellen können. Die Verschlussmittelabschnitte können dabei in beliebigen oder vorbestimmten Positionen zueinander angeordnet und verbunden werden.

Auch der erste Halteriemen kann auf eine ähnliche Weise mittels eines Verschlussmittels an jeweils einem offenen Endabschnitt des Halteriemens in einer Länge veränderbar ausgebildet sein. Auf diese Weise kann eine Anpassung der Länge des ersten Halteriemens an einen Körperumfang eines Patienten besser angepasst werden. Dies kann einen Tragkomfort erhöhen.

Die Tragevorrichtung kann zudem einen dritten Halteriemen aufweisen. Der dritte Halteriemen kann insbesondere identisch und/oder symmetrisch zu dem zweiten Halteriemen ausgebildet sein und dazu ausgelegt sein, um eine zweite Schulter eines Patienten geführt zu werden. Dies kann eine Kräfteverteilung zum Tragen einer tragbaren Gasaustauschvorrichtung verbessern, indem eine gleichmäßige Belastung beider Schultern eines Patienten ermöglicht werden kann. Dies kann auch ein ungewolltes Verrutschen der Tragevorrichtung verringern. Es versteht sich dabei, dass auch der dritte Halteriemen ein Mittel zur Anpassung dessen Länge aufweisen kann.

Die erfindungsgemäße Tragevorrichtung kann zumindest einen Verschluss zum Öffnen und Schließen wenigstens eines der Halteriemen aufweisen. Der Verschluss zum Öffnen und Schließen der Tragevorrichtung kann auch mit dem Mittel zum Verstellen einer Länge eines Halteriemens zusammenfallen. Insbesondere kann ein Verschluss zum Öffnen und Schließen des ersten Halteriemens vorgesehen sein. Auf diese Weise kann die Tragevorrichtung vereinfacht an einem Patientenkörper angelegt werden. Dies kann insbesondere dann vorteilhaft sein, wenn der Patient bereits über einen extrakorporalen Blutkreislauf mit einer tragbaren Gasaustauschvorrichtung verbunden ist, so dass die Tragevorrichtung angelegt werden kann, ohne den extrakorporalen Blutkreislauf zu beeinträchtigen.

Der wenigstens eine Verschluss kann dabei als ein Haftverschluss und/oder ein Klemmverschluss und/oder ein Eingriffsverschluss ausgebildet sein oder einen der aufgeführten Verschlüsse umfassen. Dabei ist es insbesondere auch möglich, dass der Verschluss ein flächiger Verschluss ist, also eine Mehrzahl von Verschlusselementen aufweist, die über eine bestimmte Fläche, bspw. eine Breite des mit dem Verschluss versehenen Halteriemens, aufweisen. Als Haftverschluss sollen diejenigen Verschlussarten verstanden werden, die auf einem Haftprinzip zweier Elemente beruhen, bspw. das Anhaften eines oder mehrerer Magnetpaare, oder die akkumulierte Haftwirkung in einem Klettverschluss. Als Klemmverschlüsse werden derartige Verschlüsse bezeichnet, die darauf beruhen, dass zwei Komponenten aneinander oder ineinander verklemmt werden, wie dies beispielsweise bei Druckknöpfen oder Schnallenverschlüssen der Fall ist. Als Eingriffsverschlüsse werden diejenigen Verschlüsse bezeichnet, die auf einem Eingriffsprinzip beruhen, beispielsweise bei dem Verhaken zweier Haken und/oder Ösen. Es versteht sich, dass auch Kombinationen aus derartigen Verschlüssen denkbar sind, die beispielsweise einander ergänzen oder kumulativ beispielsweise als Sicherheitsmaßnahme unabhängig voneinander vorgesehen sind. Unter die oben genannten Verschlussarten sollen im Rahmen der Erfindung dabei auch jedwede Art von Gürtelverschlüssen fallen. Es versteht sich, dass weitere Verschlussarten möglich sind, die geeignet sind, einen Halteriemen an sich selbst, an einem anderen Halteriemen oder an einem Abschnitt der Tragevorrichtung zu befestigen.

Die erfindungsgemäße Tragevorrichtung kann derart ausgebildet sein, dass der wenigstens eine Verschluss in einem geschlossenen Zustand beispielsweise den zweiten bzw. den dritten Halteriemen mit dem ersten Halteriemen verbindet. Insbesondere können auch zwei der Halteriemen einstückig miteinander ausgebildet sein, beispielsweise der zweite Halteriemen und der dritte Halteriemen. Zudem können auch alle Halteriemen einstückig miteinander ausgebildet sein. Auf diese Weise kann die Tragevorrichtung z.B. ähnlich einer Weste ausgebildet sein, mit zwei Schulterriemen, die entlang eines vertikalen Abschnitts auf einer Vorder- und/oder Hinterseite miteinander verbunden sind. Dies kann wiederum den Umgang mit der Tragevorrichtung für einen Patienten vereinfachen und die Anzahl der Verschlüsse kann reduziert werden.

Gemäß weiterer Ausführungsformen kann die Tragevorrichtung des Weiteren eine Kabelaufnahme aufweisen. Die Kabelaufnahme kann insbesondere an dem ersten Halteriemen vorgesehen sein und ist ausgebildet, wenigstens ein Kabel, insbesondere an dem ersten Halteriemen, zu halten und/oder zu sichern. Dies kann es erlauben, die Tragevorrichtung für eine tragbare Gasaustauschvorrichtung für einen Patienten sicherer zu gestalten, da Kabel, die an einer Gasaustauschvorrichtung vorgesehen sein können, sicher am Patientenkörper gehalten werden können. Die Kabelaufnahme kann dabei eine selbstschließende Aufnahme, beispielsweise eine durch Federkraft schließende Aufnahme darstellen. Die Kabelaufnahme kann dabei so dimensioniert sein, dass ein Kabel von der Kabelaufnahme locker geführt oder in der Kabelaufnahme einklemmbar ist. Durch ein Klemmen eines Kabels kann ein unbeabsichtiger Zug an dem Kabel durch die Kabelaufnahme abgeleitet werden, ohne dass das Kabel in der Kabelaufnahme verschoben oder deplatziert wird. Im Falle, dass ein Versorgungskabel der Gasaustauschvorrichtung in der Kabelaufnahme aufgenommen ist, kann auf diese Weise ein unbeabsichtigtes Herausziehen des Kabels aus der Gasaustauschvorrichtung oder die Beschädigung von mit dem Kabel verbundenen Komponenten vermieden oder zumindest verringert werden.

In alternativen Ausführungsformen kann die Kabelaufnahme auch innerhalb eines der Halteriemen, insbesondere innerhalb des ersten Halteriemens, geführt sein. "Innerhalb" soll dabei eine, in einem an einen Patientenkörper angelegten Zustand der Tragevorrichtung, dem Patientenkörper zugewandte Seite des Halteriemens sein. Es versteht sich, dass die Kabelaufnahme auch eine längliche Ausdehnung haben kann, so dass sich die Kabelaufnahme entlang des Halteriemens, insbesondere des ersten Halteriemens, erstreckt. So kann die Kabelaufnahme auch einen, innerhalb oder außerhalb des Halteriemens, verlaufenden Tunnelabschnitt bilden, in dem ein Kabel aufgenommen und geführt werden kann.

Auf diese Weise kann eine sichere Kabelführung gewährleistet werden, so dass eine Beeinträchtigung eines Patienten reduziert werden kann.

Weiterhin ist es in einigen Ausführungsformen der vorliegenden Erfindung denkbar, dass die Tragevorrichtung eine Schlauchaufnahme zur Aufnahme eines oder mehrere Schläuche aufweist. Die Schlauchaufnehme kann insbesondere zur Aufnahme eines Schlauches eines extrakorporalen Blutkreislaufs ausgebildet sein. Dabei kann die Schlauchaufnahme insbesondere derart ausgebildet sein, dass der oder die darin aufzunehmenden Schläuche an einer Innenseite der Tragevorrichtung, also an einer einem Patientenkörper zugewandten Seite, geführt werden. Auf diese Weise kann ein Wärmeverlust eines in dem oder den Schläuchen geführten Fluids, insbesondere Blut, verringert werden.

Vorzugsweise ist der erste Halteriemen der Tragevorrichtung zumindest im geschlossenen Zustand des Verschlusses nach radial außen gekrümmt, wobei der Krümmungsradius bereichsweise unterschiedlich ist. Hierdurch ist der erste Halteriemen optimal an eine Positionierung auf der Taille eines Patienten angepasst.

Ein zweiter Aspekt der vorliegenden Erfindung betrifft ein Tragesystem, das eine Tragevorrichtung, insbesondere gemäß des ersten Aspekts der vorliegenden Erfindung, sowie eine tragbare Gasaustauschvorrichtung aufweist. Die Gasaustauschvorrichtung weist dabei ein Befestigungsmittel auf, das komplementär zu einem Befestigungsmittel der Tragevorrichtung ist, so dass die Gasaustauschvorrichtung, insbesondere reversibel, an der Tragevorrichtung befestigt werden kann. Ein derartiges Tragesystem kann es einem Patienten erlauben, sich mobil, zumindest in einem begrenzten Radius und unabhängig von stationären Systemen, zu bewegen.

Die Gasaustauschvorrichtung des Tragesystems kann eine Steuereinheit aufweisen. Die Steuereinheit kann wiederum ein Befestigungsmittel aufweisen, das zur Befestigung an der Tragevorrichtung, insbesondere an einem der Riemen, bspw. dem ersten Halteriemen der Tragevorrichtung, und/oder an der Gasaustauschvorrichtung ausgebildet ist. Auf diese Weise kann die Steuereinheit in unmittelbarer Nähe der Gasaustauschvorrichtung sicher gelagert werden und kann einem Patienten oder einer Hilfsperson einen schnellen Zugriff auf die Steuereinheit und die Funktionen der Gasaustauschvorrichtung ermöglichen.

In einigen Ausführungsformen der vorliegenden Erfindung kann die Steuereinheit auch direkt in die Gasaustauschvorrichtung integriert ausgebildet sein.

Die Gasaustauschvorrichtung kann zudem wenigstens ein Kabel zur Verbindung mit der Steuereinheit aufweisen, wobei das Kabel in einer an der Tragevorrichtung ausgebildeten Kabelaufnahme aufgenommen sein kann. Dies kann eine sichere Verstauung des Kabels erlauben, und somit eine Beeinträchtigung eines Patienten reduzieren. Es versteht sich, dass die Steuereinheit und die tragbare Gasaustauschvorrichtung alternativ auch kabellos miteinander verbunden sein können. So kann beispielsweise eine Funkverbindung, eine Bluetooth-Verbindung, eine Infrarotverbindung oder weitere drahtlose Übertragungsarten und Übertragungsprotokolle verwendet werden, um die Steuereinheit mit der Gasaustauschvorrichtung zu verbinden. Dies kann die Verwendung eines Kabels umgehen und somit das Gewicht des Tragesystems verringern und den Tragekomfort des Tragesystems erhöhen.

Grundsätzlich kann die Kabelaufnahme des Tragesystems ausgebildet sein, wie bereits für die Tragevorrichtung beschrieben, insbesondere also auch als eine langgestreckte Aufnahme, um ein Kabel eine vorbestimmte Länge entlang eines Patientenkörpers zu leiten.

An der Steuereinheit kann des Weiteren ein Mittel vorgesehen sein, das eine Befestigung der Steuereinheit an der tragbaren Gasaustauschvorrichtung und/oder an der Tragevorrichtung erlaubt. An der Gasaustauschvorrichtung und/oder an der Tragevorrichtung kann dazu ein komplementäres Mittel vorgesehen werden, so dass beispielsweise ein Befestigungsabschnitt der Steuereinheit mit einem Befestigungsabschnitt der Gasaustauschvorrichtung in Eingriff geraten und eine zuverlässige Befestigung der Gasaustauschvorrichtung ermöglichen kann. Dazu kann in einigen Ausführungsformen an einem der Halteriemen, insbesondere an dem ersten Halteriemen, eine Lasche ausgebildet sein, die zur Aufnahme des Befestigungsabschnitts der Steuereinheit ausgebildet und dimensioniert ist.

Die Befestigung der Steuereinheit kann beispielsweise an dem Gehäuse der Gasaustauschvorrichtung vorgesehen sein. Alternativ kann eine Befestigung der Steuereinheit auch an einem Tragesystem der Gasaustauschvorrichtung vorgesehen sein. Zudem kann eine Befestigung der Steuereinheit auch unmittelbar an der Kleidung eines Patienten erfolgen. Zudem kann das Befestigungsmittel der Steuereinheit auch dazu vorgesehen sein, die Steuereinheit an einer Tragestruktur, beispielsweise an einem Patientenbett oder einem Tragesystem für medizinische Geräte in einem Krankenzimmer zu befestigen.

Insbesondere kann das Befestigungsmittel der Steuereinheit eine hakenförmige Ausbildung haben, die ein Einhaken des Befestigungsmittels an einem komplementären Aufnahmeabschnitt oder beispielsweise an einem rohrförmigen Element oder einer Außenkontur des Gehäuses der Gasaustauschvorrichtung ermöglicht. Zudem ist eine Befestigung an einer flachen Struktur, wie beispielsweise einer Rollstuhllehne oder ähnlichem denkbar. Dazu kann das Befestigungsmittel der Steuereinheit beispielsweise mit einem unter einer Federvorspannung stehenden Federarm ausgebildet sein, so dass eine Klemmung aufgrund der Federvorspannung des Befestigungsmittels eine ausreichende Haltekraft zur Befestigung der Steuereinheit bereitstellt. Im Übrigen kann das Befestigungsmittel oder ein zusätzliches Standmittel derart ausgebildet und an der Steuereinheit ausgebildet sein, dass die Steuereinheit auf einer Unterlage, beispielsweise einem Tisch oder einem Rollcontainer oder ähnlichem, aufgestellt werden kann.

Im Übrigen ist es denkbar, dass die Tragevorrichtung des Tragesystems mit einem integrierten Kabel ausgebildet ist, das zur Verbindung der Steuereinheit mit der Gasaustauschvorrichtung ausgebildet ist. Dabei kann die Tragevorrichtung, beispielsweise an einem Halteriemen, mit entsprechenden Anschlüssen ausgebildet sein. Ein Anschluss kann dabei beispielsweise auch integral mit einem Befestigungsmittel zum Befestigen der Gasaustauschvorrichtung an der Tragevorrichtung oder einem Befestigungsmittel zum Befestigen der Steuereinheit an der Tragevorrichtung oder der Gasaustauschvorrichtung ausgebildet sein. Eine "integrale" Ausbildung soll dabei auch diejenigen Ausführungen der Erfindung umfassen, in denen ein Befestigungsmittel und eine Verbindung, insbesondere eine elektrische Verbindung, zur Verbindung mit der Steuereinheit lokal getrennt, jedoch miteinander gekoppelt vorgenommen werden. Dies betrifft insbesondere diejenigen Fälle, in denen durch das Befestigen der Gasaustauschvorrichtung mit der Tragevorrichtung auch unmittelbar eine, insbesondere elektrische, Verbindung mit der Tragevorrichtung und/oder mit der Steuereinheit erfolgt.

Die Gasaustauschvorrichtung, die insbesondere eine Vorrichtung zur Lungenunterstützung sein kann, besteht dabei im Wesentlichen aus einem Gehäuse, einem Pumpensystem und einem Gasaustauschmittel. Das Gasaustauschmittel ist dabei vorzugsweise gemeinsam mit dem Pumpensystem in dem Gehäuse der Gasaustauschvorrichtung aufgenommen. Dies ermöglicht es, eine kompakte und leicht am Körper tragbare Gasaustauschvorrichtung bereitzustellen, die von einem Patienten oder Hilfspersonen leicht, sicher und zuverlässig nahe an bzw. unmittelbar an einem Patientenkörper zu befestigen. Dies kann die Bewegungsfreiheit und den Tragekomfort für einen Patienten erhöhen. Im Übrigen kann eine derartige Gasaustauschvorrichtung die Sicherheit für einen Patienten erhöhen, da der kompakte und integrierte Aufbau der Gasaustauschvorrichtung externe Zugänge zu der Gasaustauschvorrichtung reduzieren kann.

Im Übrigen ist es im Rahmen der Erfindung denkbar, dass die Steuereinheit zumindest teilweise in die Tragevorrichtung integriert ausgebildet ist. So kann beispielsweise eine Anzeigeeinheit zum Anzeigen relevanter Parameter der Gasaustauschvorrichtung an bzw. auf der Tragevorrichtung ausgebildet sein. Zudem ist es denkbar, dass die Tragevorrichtung, die Gasaustauschvorrichtung und/oder die Steuereinheit einen Alarmknopf und/oder eine Mobilfunkvorrichtung aufweisen, insbesondere zum Herstellen einer manuellen oder automatischen Notrufverbindung im Falle einer Fehlfunktion der Gasaustauschvorrichtung.

Im Übrigen ist zu beachten, dass die verschiedenen Ausführungsformen der vorliegenden Erfindung insbesondere auf eine Vorrichtung zur Lungenunterstützung zutreffen, also eine Vorrichtung, die eine Lungenfunktion nur teilweise erfüllt. Insbesondere dadurch, dass die Lungenfunktion unterstützt wird, beispielsweise durch Reduzierung der CO₂-Konzentration in Blut, jedoch nicht gänzlich ersetzt wird, kann eine tragbare, kompakte und hoch-mobile Gasaustauschvorrichtung bereitgestellt werden, da keine schweren Gasflaschen, beispielsweise Sauerstoffflaschen, mittransportiert werden müssen.

Entsprechend weisen einige Ausführungsformen des erfindungsgemäßen Tragesystems einen Umgebungsluftansaugabschnitt auf. Der Umgebungsluftansaugabschnitt kann insbesondere an der Gasaustauschvorrichtung des Tragesystems vorgesehen sein. Dieser Umgebungsluftansaugabschnitt kann dabei einen oder mehrere Filter zum Filtern der Umgebungsluft aufweisen. Die angesaugte Umgebungsluft kann dann, beispielsweise mittels eines Schlauchsystems, zu einem Gasaustauschabschnitt der Gasaustauschvorrichtung geleitet werden. Insbesondere bei der Verwendung zur Lungenunterstützung durch CO₂ Reduktion im Blut ist es ausreichend, Umgebungsluft, also normale Atemluft, die einen CO₂ Gehalt von in der Regel weniger als 0,05% aufweist, zur Abreicherung von CO₂ aus dem Blut zu verwenden. Die CO₂ Reduktion kann dabei unabhängig von einer Oxygenierung des Blutes erfolgen, wie dies beispielsweise im Falle von COPD der Fall sein kann. Das Mitführen von Sauerstoffflaschen oder anderen Gasflaschen und der damit verbundene Transport der schweren Druckbehälter, ist daher nicht zwangsläufig notwendig. Somit wird ermöglicht, dass auch ein körperlich eingeschränkter Patient das erfindungsgemäße Tragesystem selbständig tragen kann, was bei einem Mitführen von schweren Druckbehältern nicht möglich ist.

Bevorzugte Ausführungsformen der Erfindung betreffen daher ein Tragesystem das kein externes Gasüberdruckelement, wie es beispielsweise eine Gaskompressionsflasche wie eine Sauerstoffflasche darstellt, aufweist. Erfindungsgemäß ist das Tragesystem bestehend aus der Tragevorrichtung und der Gasaustauschvorrichtung ohne eine externe Gasversorgung, nur mittels eines pumpenbetriebenen Umgebungsluftansaugabschnitts zur Lungenunterstützung geeignet.

Vorteilhaft weist die Gasaustauschvorrichtung ein Gehäuse auf, das eine dem ersten Halteriemen zugewandte Rückwand aufweist, die zum ersten Halteriemen hin nach innen gekrümmt ist, wodurch das Gehäuse nah am Halteriemen geführt werden kann, so dass der Schwerpunkt der Gasaustauschvorrichtung bei deren Verwendung nahe am Körper eines Patienten zu liegen kommt, was den Tragekomfort erhöht und die Belastung des Patienten auf Grund eines geringen Hebels minimiert.

In einer günstigen Weiterbildung, korrespondiert die Krümmung der Rückwand, mit der Krümmung des ersten Halteriemens im Wesentlichen, so dass die Rückwand des Gehäuses im Wesentlichen bündig an dem nach radial außen gekrümmten ersten Halteriemen anliegt, wodurch der Tragekomfort weiter optimiert ist.

Weiterhin von Vorteil ist es, wenn das Gehäuse eine durchschnittliche Breite, eine senkrecht zu dieser Breite verlaufende durchschnittliche Höhe und eine senkrecht zur Breite und zur Höhe verlaufende durchschnittliche Tiefe aufweist, wobei sich die Tiefe von der Rückwand weg erstreckt und wobei die Tiefe eine maximale Größe von 20%, vorzugsweise 15%, der summarischen Gesamtlänge aus Breite + Höhe + Tiefe aufweist. Durch diese Maßnahme erhält die Gasaustauschvorrichtung eine flache Gestalt, die im Anwendungszustand nur einen sehr kleinen Hebel auf den Patienten ausübt, so dass die Gasaustauschvorrichtung über längere Zeit getragen werden kann, ohne den Patienten zu ermüden.

Im Übrigen versteht es sich, dass die erfindungsgemäße Vorrichtung bzw. das erfindungsgemäße System derart ausgebildet sein kann, dass sie die allgemeine Richtlinie für Medizinprodukte 93/42/EWG oder ähnliche Vorgaben erfüllen.

Die Erfindung sowie vorteilhafte Weiterbildungen davon werden im Folgenden anhand bestimmter in den beigefügten Zeichnungen dargestellter Ausführungsbeispiele erläutert, in denen gleiche Merkmale mit den gleichen Bezugszeichen versehen sind. Es zeigt:
- Figur 1: zeigt eine Vorderansicht einer Tragevorrichtung gemäß einer Ausführungsform der vorliegenden Erfindung;
- Figur 2: zeigt die Tragevorrichtung nach Figur 1 mit einer tragbaren Gasaustauschvorrichtung;
- Figur 3: zeigt die Tragevorrichtung nach Figur 1 und 2 mit einer Steuereinheit;
- Figur 4: zeigt eine Tragevorrichtung gemäß einer weiteren Ausführungsform der vorliegenden Erfindung;
- Figur 5: zeigt eine Tragevorrichtung gemäß einer weiteren Ausführungsform der vorliegenden Erfindung;
- Fig. 6: zeigt ein Tragesystem beinhaltend eine nur angedeutete Tragevorrichtung und eine Gasaustauschvorrichtung von der nur ein Gehäuse wiedergegeben ist;
- Fig. 7: zeigt ein Gehäuse der Gasaustauschvorrichtung und einen Halteriemen der Tragevorrichtung des Tragesystems aus Fig. 6 in einem Schnitt entlang der Linie VII-VII aus Fig. 6;
- Fig. 8: zeigt eine Tragevorrichtung gemäß einer weiteren Ausführungsform der vorliegenden Erfindung;
- Fig. 9: zeigt eine Tragevorrichtung gemäß einer weiteren Ausführungsform der vorliegenden Erfindung.

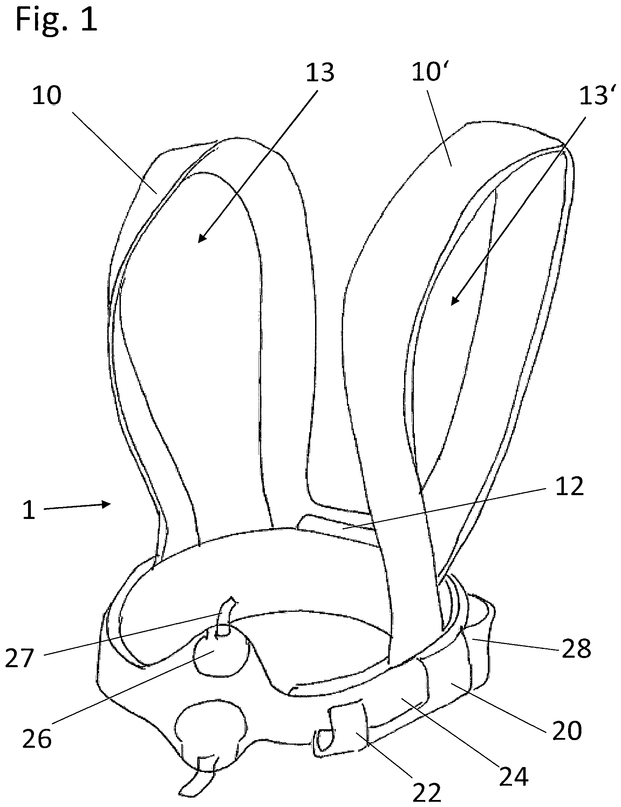

Figur 1 zeigt eine perspektivische Vorderansicht einer Tragevorrichtung 1 gemäß einer Ausführungsform der vorliegenden Erfindung.

Hier und im Folgenden soll zur vereinfachten Beschreibung der vorliegenden Erfindung eine Richtungsangabe gelten, wie sie beim bestimmungsgemäßen Tragen der Tragevorrichtung 1 von einem Patienten verwendet werden würde. Als "oben" wird demnach ein Abschnitt bezeichnet, der einem Kopf bzw. einer Schulter eines Patienten zugewandt ist oder nahe liegt. Entsprechend wird eine Bezeichnung "unten" für Abschnitte oder Komponenten der Tragevorrichtung 1 verwendet, die in einem den Füßen eines Patienten zugewandten Bereich der Tragevorrichtung 1 angeordnet oder vorgesehen sind. Eine Richtung von unten nach oben, oder anders herum, wird als eine vertikale Richtung bezeichnet. Eine zu der vertikalen Richtung senkrechte Richtung, die insbesondere eine Umfangsrichtung um einen Torso bzw. eine Hüfte oder Taille eines Patienten beschreiben soll, wird auch als eine waagerechte Richtung oder Ausrichtung bezeichnet. Eine "vordere" Position beschreibt analog eine Position, die eine Vorderseite eines Trägers der Tragevorrichtung 1 beschreibt. Eine "hintere" Position beschreibt entsprechend eine Seite, die einer Rückseite bzw. einer Seite des Rückens eines Trägers der Tragevorrichtung 1 entsprechen würde.

Die Tragevorrichtung 1 gemäß Figur 1 weist entsprechend der obigen Definitionen einen ersten Halteriemen 20 auf. Der erste Halteriemen 20 verläuft in einer waagerechten Richtung und ist dazu vorgesehen, um einen Torso bzw. eine Hüfte eines Patienten geführt zu werden. Der erste Halteriemen 20 bildet insofern im Wesentlichen einen Ring, der von einer Vorderseite um einen seitlichen Abschnitt über eine Rückseite und einen zweiten seitlichen Abschnitt eines Patienten wieder zur Vorderseite geführt ist.

Ein zweiter Haltriemen 10 und ein dritter Halteriemen 10' weisen jeweils einen ersten Schenkel 11a und einen zweiten Schenkel 11b auf. Der zweite Halteriemen 10 und der dritte Halteriemen 10' bzw. die entsprechenden Schenkel 11a und 11b weisen einen im Wesentlichen vertikalen Verlauf auf. Die Schenkel 11a und 11b sind in der in Figur 1 gezeigten Ausführungsform einstückig ausgebildet, so dass die Trageriemen 10, 10' an einem oberen Abschnitt ineinander übergehen und jeweils eine Schlaufe bilden. Zumindest einer der Schenkel 11a ist dabei mit einem Ende an dem ersten Halteriemen 20 befestigt. Von dem Befestigungspunkt an dem ersten Riemen 20 erstreckt sich der Schenkel 11a in einer vertikalen Richtung und geht an einem obersten Abschnitt in den zweiten Schenkel 11b über. Der zweite Schenkel 11b des Halteriemens 10 kann ebenfalls an dem ersten Halteriemen 20 befestigt sein. In anderen Ausführungsformen der vorliegenden Erfindung ist ein unteres Ende des zweiten Schenkels 11b des zweiten Halteriemens 10 mit einem unteren Ende eines Schenkels des dritten Halteriemens 10' verbunden. Die Verbindung ist mittels eines Verbindungsstegs hergestellt. An dem Verbindungssteg ist ein Verschlussmittel 12 vorgesehen.

In dem gezeigten Ausführungsbeispiel der Figur 1 ist das Verschlussmittel 12 ein Klettverschluss. Ein entsprechendes Gegenstück des Verschlussmittels 12 ist gemäß Figur 1 an dem zweiten Halteriemen 20 derart vorgesehen, dass das Verschlussmittel 12 sicher an dem zweiten Halteriemen 20 befestigt werden kann. Auf diese Weise erfolgt eine Befestigung des zweiten Schenkels 11b des ersten Halteriemens 10 bzw. des dritten Halteriemens 10' an dem ersten Halteriemen 20.

Das Verschlussmittel 12 des zweiten Halteriemens 10 bzw. des dritten Halteriemens 10' weist ein entsprechendes Gegenstück des Verschlussmittels 12 auf, das mit dem Verschlussmittel 12 zusammenwirkt. Das Gegenstück des Verschlussmittels ist dabei an dem Rückabschnitt des ersten Halteriemens ausgebildet, so dass eine Verbindung zwischen dem ersten Halteriemen 20 und dem zweiten Halteriemen 10 und dem dritten Halteriemen 10' hergestellt werden kann.

Der zweite Halteriemen 10 und der dritte Halteriemen 10' weisen gemäß der in Figur 1 dargestellten Ausführungsform somit grundsätzlich eine U-Form mit einer nach unten gerichteten Öffnung auf, die in einem verschlossenen Zustand, also einem Zustand in dem die Tragevorrichtung korrekt angelegt ist, von dem ersten Halteriemen 20 begrenzt wird. Die durch die Halteriemen 10, 10' gebildeten Schlaufen stellen jeweils eine Öffnung 13, 13' dar, durch die jeweils ein Arm eines Patienten geführt werden kann.

Die Befestigung des ersten Schenkels 11a des zweiten Halteriemens 10 bzw. 10' erfolgt in der gezeigten Ausführungsform nach Figur 1 an einem seitlichen, vorderen Abschnitt des ersten Halteriemens 20. Der entsprechend zweite Schenkel 11b des zweiten Halteriemens 10 bzw. 10' ist zu einem rückwärtigen Abschnitt, d.h. einem Rückenbereich eines Patienten im angelegten Zustand, geführt.

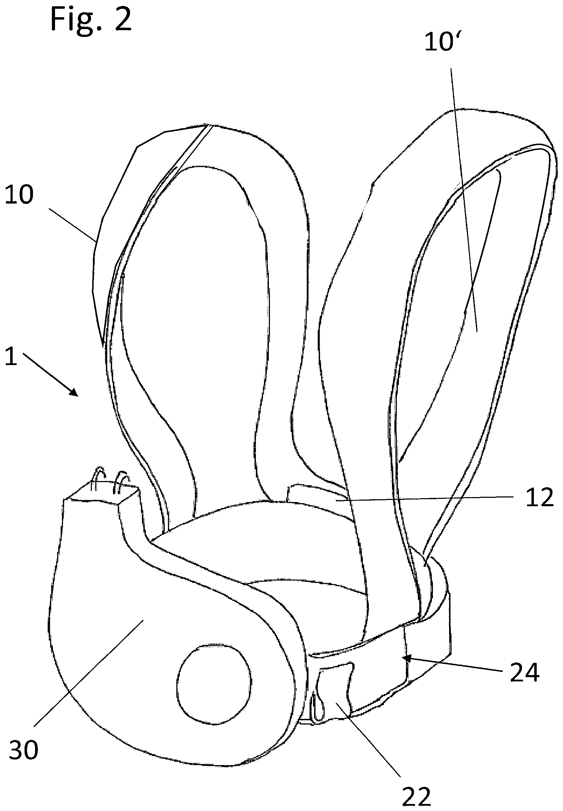

Der erste Halteriemen 20 weist zudem einen zweiten Verschluss 24 auf. Der zweite Verschluss 24 kann auf ähnliche Weise als Klettverschluss ausgebildet sein, wie das Verschlussmittel 12. Der zweite Verschluss 24 ist in einer Umfangsrichtung des ersten Halteriemens 20 ausgebildet. Somit ermöglicht der zweite Verschluss 24 ein Anlegen des ersten Halteriemens 20 um den Körper eines Patienten und das Anpassen einer Länge des ersten Halteriemens 20. Der zweite Verschluss 24 ist in der gezeigten Ausführungsform nach Figur 1 zudem teilweise als Teil eines Befestigungsmittels 26 ausgebildet. Das Befestigungsmittel 26 dient der Aufnahme einer tragbaren Gasaustauschvorrichtung 30, wie in Figur 2 gezeigt ist. Dazu weist das Befestigungsmittel 26 in der Ausführungsform nach Figur 1 eine Aufnahme zur Aufnahme eines an der Gasaustauschvorrichtung 30 befestigten Vorsprungs oder Einrastmittels auf.

Beim Einführen des Einrastmittels der Gasaustauschvorrichtung 30 in die Aufnahme des Befestigungsmittels 26 kann dabei ein Einrastmechanismus, der beispielsweise federvorgespannt in der Aufnahme des Befestigungsmittels 26 ausgebildet ist, verdrängt werden, so dass das Einrastmittel der Gasaustauschvorrichtung 30 tiefer in die Aufnahme des Befestigungsmittels 26 eindringen kann. Durch Vorsehen einer Einkerbung oder eines Widerhakens an dem Einrastmittel der Gasaustauschvorrichtung 30 kann bei entsprechendem Einbringen des Einrastmittels in die Aufnahme des Befestigungsmittels 26 das Eingriffsmittel in der Aufnahme in die Einkerbung eingreifen und somit das Einrastmittel der Gasaustauschvorrichtung 30 aufgrund des durch die Federspannung eingreifenden Eingriffsmittels an der Tragevorrichtung 1 sichern. An dem Befestigungsmittel 26 kann zudem ein mit dem Eingriffsmittel verbundenes Auslösemittel, hier eine Lasche 27, ausgebildet sein, die ein Lösen des Eingriffs des Eingriffsmittels mit dem Vorsprung der Gasaustauschvorrichtung 30 bewirkt. Das Auslösemittel kann auch auf andere Weise als mittels einer Lasche ausgebildet sein.

Das Befestigungsmittel 26 der Tragevorrichtung 1 weist in der Ausführungsform der Figur 1 zwei derartige in einer vertikalen Richtung versetzte Aufnahmen auf. Dies kann einen verbesserten Halt sowie ein Verkippen oder Verrutschen der tragbaren Gasaustauschvorrichtung 30 bewirken bzw. reduzieren. Die Laschen 27 zum Lösen des Eingriffsmittels in dem Befestigungsmittel 26 sind dabei in entgegengesetzte Richtungen, bei der oberen Aufnahme nach oben und bei der unteren Aufnahme nach unten ausgebildet. Dies kann einen erleichterten Zugriff beim Entfernen der Gasaustauschvorrichtung 30 von der Tragevorrichtung ermöglichen. Die Aufnahmen können auch horizontal oder auf andere Weise zueinander versetzt angeordnet sein.

An dem ersten Halteriemen 20 ist zudem eine Kabelaufnahme 22 ausgebildet. Das Befestigungsmittel 26 ist an einem vorderen Bereich, also entsprechend an einer Vorderseite eines Patienten, ausgebildet. Die Kabelaufnahme 22 ist in einer seitlichen Richtung versetzt benachbart zu dem Befestigungsmittel 26 ausgebildet.

Die Kabelaufnahme 22 kann beispielsweise als ein flexibles bzw. biegsames Element ausgebildet sein, das aufgrund einer inhärenten Federvorspannung selbstschließend ist, also ähnlich einer Klammer an dem Halteriemen 20 mit einem Schenkel anliegt. Die Kabelaufnahme 22 ist in einer Querschnittsform tropfenförmig ausgebildet, also geformt, einen Hohlraum zu umschließen. Dieser von der Kabelaufnahme 22 geformte Hohlraum ist so dimensioniert, dass wenigstens ein Kabel einer vorbestimmten Stärke darin aufgenommen werden kann.

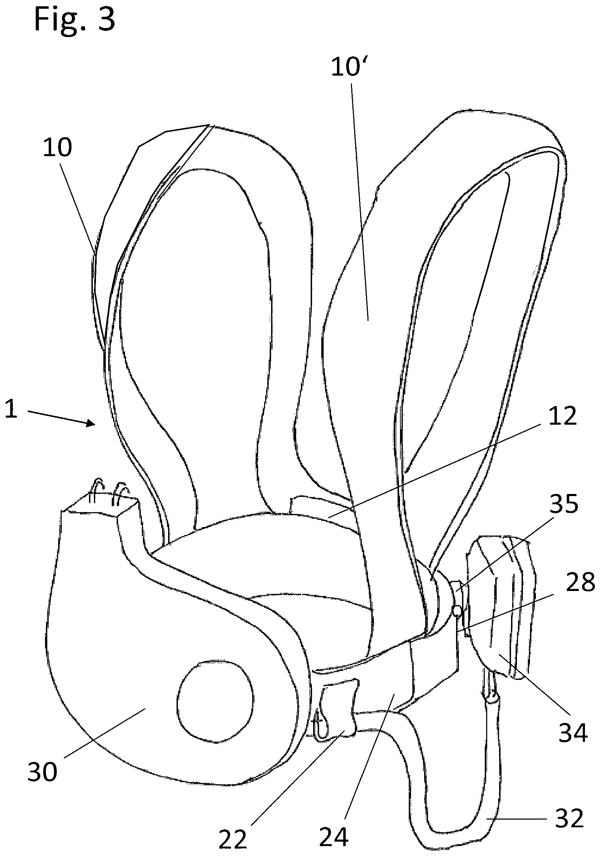

Im Übrigen weist der erste Halteriemen 20 gemäß der in Figur 1 dargestellten Ausführungsform in einem seitlich hinteren Abschnitt des Halteriemens 20 eine Schlaufe 28 auf. Die Schlaufe 28 kann dabei insbesondere durch einen an dem Halteriemen 20 befestigten Stoffabschnitt gebildet sein. Die Schlaufe 28 ist dabei derart dimensioniert, dass eine Aufnahme beispielsweise eines Klammerabschnitts, d.h. eines flächigen Streifens, ausgebildet ist. Insbesondere, wie der Figur 3 zu entnehmen ist, kann die Schlaufe 28 zur Aufnahme eines Halterclips einer Steuereinheit 34 dienen.

Figur 2 zeigt die Tragevorrichtung 1 gemäß Figur 1, wobei an einer Vorderseite der Tragevorrichtung 1, d.h. an einer Vorderseite des ersten Halteriemens 20, eine tragbare Gasaustauschvorrichtung 30 angeordnet ist. Es versteht sich, dass die tragbare Gasaustauschvorrichtung 30 über wenigstens ein Befestigungsmittel-Gegenstück an dem Befestigungsmittel 26 der Tragevorrichtung 1 befestigt ist. Dabei ist das Befestigungsmittel-Gegenstück insbesondere ein Vorsprung bzw. ein Einrastmittel. Vorzugsweise weist die Gasaustauschvorrichtung 30 zwei entsprechende, mit dem Befestigungsmittel 26 der Tragevorrichtung 1 korrespondierende Einrastmittel zum Einbringen in die Aufnahmen des Befestigungsmittels 26 auf.

In den Figuren ist hier, wie auch im Folgenden, eine konsistente Bezeichnung von gleichen bzw. gleichwirkenden Komponenten verwendet, deren Beschreibung nicht wiederholt wird.

Figur 3 zeigt eine Tragevorrichtung 1 gemäß den Figuren 1 und 2, wobei zusätzlich zu der tragbaren Gasaustauschvorrichtung eine Steuereinheit 34 zur Steuerung der tragbaren Gasaustauschvorrichtung 30 dargestellt ist. Die Steuereinheit 34 weist einen Befestigungsabschnitt 35 auf. Der Befestigungsabschnitt 35 weist dabei einen flächigen Streifen, insbesondere einen Metallstreifen, auf, der zur Aufnahme in der Schlaufe 28 des zweiten Halteriemens 20 vorgesehen ist. Die Breite des Flächenabschnitts des Halteabschnitts 35 und die Breite bzw. Dimensionierung der Schlaufe 28 sind dabei aufeinander abgestimmt, so dass die Steuereinheit 34 sicher in der Schlaufe 28 gehalten werden kann. Die Ausrichtung der Schlaufe 28 kann dabei, wie gezeigt, in vertikaler Richtung vorgesehen sein. Alternativ kann die Ausrichtung der Schlaufe 28, und entsprechend die Ausrichtung des Halteabschnitts 35 der Steuereinheit 34, in einer horizontalen Ausrichtung, also in Umfangsrichtung, vorgesehen sein.

Zudem zeigt Figur 3 eine Verbindung zwischen der tragbaren Gasaustauschvorrichtung 30 und der Steuereinheit 34 mittels eines Kabels 32. Das Kabel 32 ist dabei aus der tragbaren Gasaustauschvorrichtung 30 durch die Kabelaufnahme 22 geführt und verläuft anschließend weiter zu der Steuereinheit 34. Durch das Vorsehen der Kabelaufnahme 22 kann das Kabel 32 sicher entlang des ersten Halteriemens 20 geführt werden. Insbesondere kann die Kabelaufnahme 22 derart dimensioniert sein, dass das Kabel 32 in der Kabelaufnahme 22 eingeklemmt wird. Auf diese Weise kann ermöglicht werden, dass das Kabel 32 bzw. die Kabelanschlüsse in der tragbaren Gasaustauschvorrichtung 30 durch unbeabsichtigten Zug nicht aus der Gasaustauschvorrichtung 30 oder der Steuereinheit 34 gelöst werden.

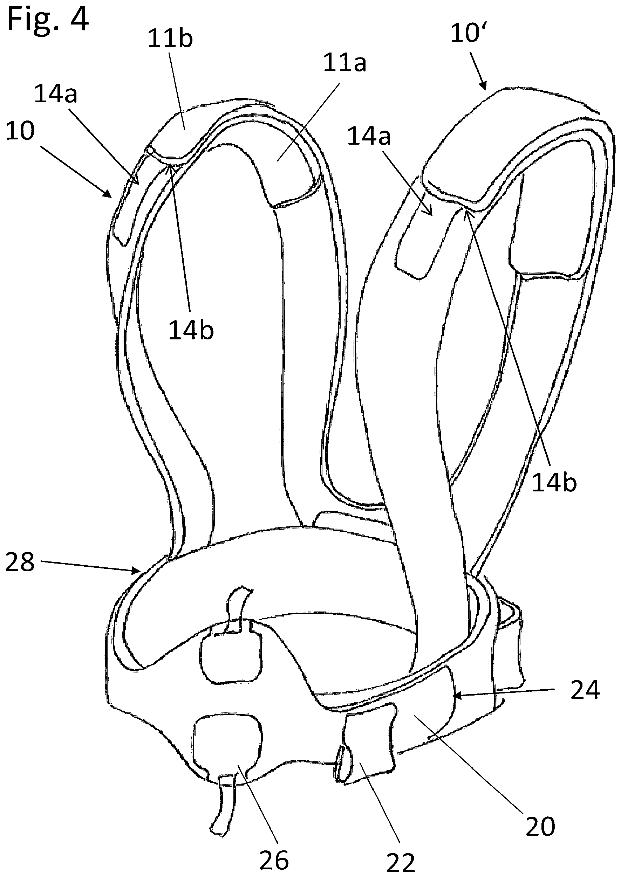

Figur 4 zeigt eine weitere Ausführungsform einer Tragevorrichtung 1 gemäß der vorliegenden Erfindung. Die Ausführungsform der Figur 4 unterscheidet sich dabei von der Ausführungsform der Figur 1 darin, dass zwei separate, voneinander getrennte Halteriemen, nämlich der zweite Halteriemen 10 und der dritte Halteriemen 10' vorgesehen sind. Die Halteriemen 10 bzw. 10' sind dabei mit dem jeweils ersten Schenkel 11a ähnlich der Ausführungsform nach Figur 1 an dem ersten Halteriemen 20 befestigt. Der zweite Schenkel 11b der Halteriemen 10, 10' ist jedoch an dem ersten Schenkel 11a des jeweiligen Halteriemens befestigt, so dass die Halteriemen jeweils eine Ring- oder O-Form aufweisen. Dabei ist an den Halteriemen ein Verstellmittel 14a, 14b vorgesehen. Das Verstellmittel 14a, 14b dient zum Verändern der Länge des jeweiligen Halteriemens und erlaubt damit eine Anpassung der Tragevorrichtung 1 an die Physiologie eines Trägers.

Dazu ist in der gezeigten Ausführungsform der erster Schenkel 11a des Halteriemens, hier beispielhaft an dem zweiten Halteriemen 10 beschrieben, mit einem ersten Verstell- bzw. Verschlussmittel 14a an einem offenen Endabschnitt des Schenkels 11a vorgesehen. Das Verstell- bzw. Verschlussmittel 14a ist in der gezeigten Ausführungsform wiederum als ein Teil eines Klettverschlusses ausgebildet, der flächig entlang des Endabschnitts des ersten Schenkels 11a des zweiten Halteriemens 10 ausgebildet ist. An einem offenen Ende des zweiten Schenkels 11b ist an einem freien Endabschnitt ein zu dem ersten Verschlussmittel 14a komplementäres Verstell- bzw. Verschlussmittel 14b vorgesehen, das wiederum flächig entlang des Endabschnitts 11b ausgebildet ist und einen zweiten Teil eines Klettverschlusses bildet. Auf diese Weise wird eine sichere flächige Verbindung zwischen den Schenkeln 11a und 11b des zweiten Halteriemens 10 ermöglicht. Analog ist ein derartiger Verschluss auch an dem dritten Halteriemen 10' ausgebildet.

In der Ausführungsform nach Figur 4 ist zudem der Verschluss 24 in einem hinteren Bereich des ersten Halteriemens 20 vorgesehen. Zudem ist die Position der Schlaufe 28 in der Ausführungsform nach Figur 4 derart verändert, dass die Schlaufe 28 in einem seitlich-vorderen Bereich des ersten Halteriemens 20 ausgebildet ist.

Es versteht sich, dass die Position der Verschlüsse, der Schlaufe oder weiterer Komponenten frei wählbar und nicht einschränkend für den erfindungsgemäßen Gedanken ist, eine Tragevorrichtung für eine tragbare Gasaustauschvorrichtung bzw. ein erfindungsgemäßes Tragesystem zur Verfügung zu stellen.

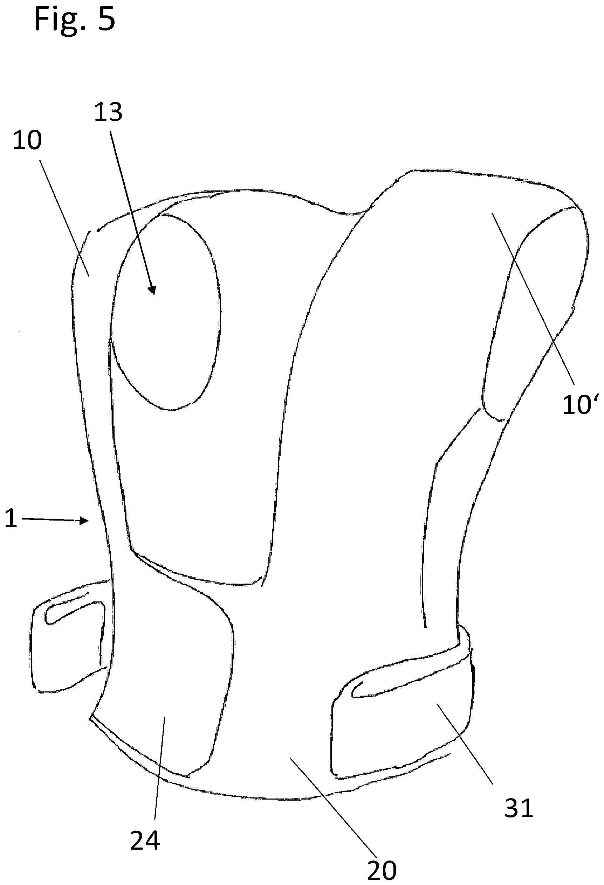

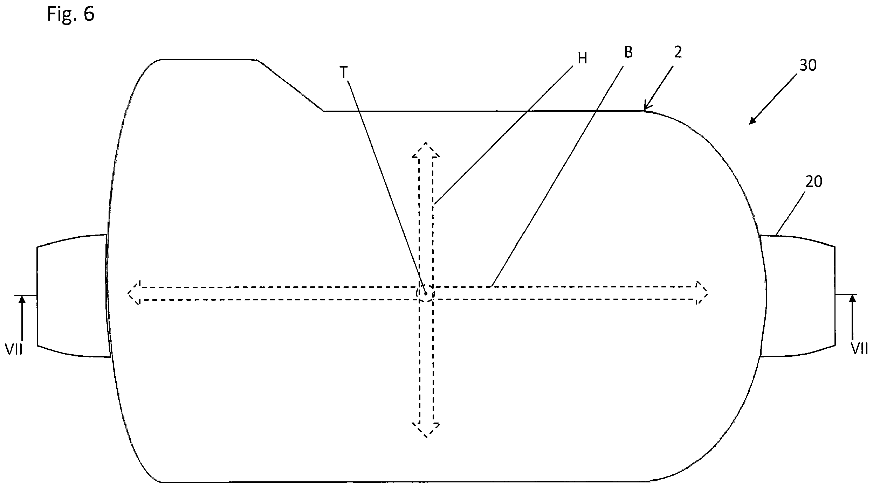

Entsprechend zeigt Figur 5 eine weitere Ausführungsform der vorliegenden Erfindung, wobei der erste Halteriemen 20, der zweite Halteriemen 10 und der dritte Halteriemen 10' einstückig miteinander ausgebildet sind. Dabei ist die Tragevorrichtung 1 derart ausgebildet, dass sie ähnlich einer Weste von einem Patienten getragen werden kann. Die Tragevorrichtung 1 ist dabei derart gestaltet, dass ein flächiger Halt, insbesondere in einem Schulterbereich und einem Rückenbereich wie auch in einem Bauchbereich eines Patienten, ermöglicht ist. Der Verschluss 24 ist in der Ausführungsform nach Figur 6 in einem vorderen Bereich der Tragevorrichtung 1 vorgesehen. Im Übrigen weist die Ausführungsform nach Figur 5 ein zusätzliches Gurtmittel 31 auf, das zusätzlich um den Torso, die Hüfte oder die Taille eines Trägers führbar ist und zur Befestigung der tragbaren Gasaustauschvorrichtung 30 vorgesehen ist. Der Gürtel 31 kann zudem zur Aufnahme der Steuereinheit 34 und/oder des Kabels 32 ausgebildet sein. Die Gasaustauschvorrichtung 30 kann in dieser Ausführungsform wiederum mit einem in Figur 5 nicht gezeigten Befestigungsmittel 26 an der Tragevorrichtung 1 befestigt werden, wobei das Befestigungsmittel mittels des Gürtels 31 gehalten werden kann bzw. an dem Gürtel 31 befestigt werden kann.

Vorzugsweise, wie nicht in Figur 5 zu erkennen ist, weist die Tragevorrichtung 1 gemäß einer Ausführungsform nach Figur 5 in einem Bereich, in dem der Gürtel 31 um die Tragevorrichtung 1 bzw. um einen Träger geführt ist, wiederum einen ersten Teil eines vorzugsweise flächigen Verschlussmittels, wie beispielsweise ein Klettverschluss, auf. Analog kann der Gürtel 31 einen entsprechenden komplementären Teil des Verschlussmittels aufweisen. So kann eine feste, vorzugsweise flächige, Verbindung zwischen dem Gürtel 31 und der Tragevorrichtung ermöglicht sein. Anstelle eines Verschlussmittels kann auch ein Reibungsmittel, wie bspw. eine Gummierung oder ähnliches vorgesehen sein, was ein Verrutschen des Gürtels 31 in einem angelegten Zustand entlang der Tragevorrichtung 1 verhindert. So kann auf einfache Weise durch Verschließen des Gurts 31 ein Verrutschen der an dem Gurt 31 befestigbaren Gasaustauschvorrichtung 31 verhindert oder reduziert werden und die Last durch die Gasaustauschvorrichtung kann von den Schultern eines Patienten aufgenommen und auf den gesamten Körper verteilt werden.

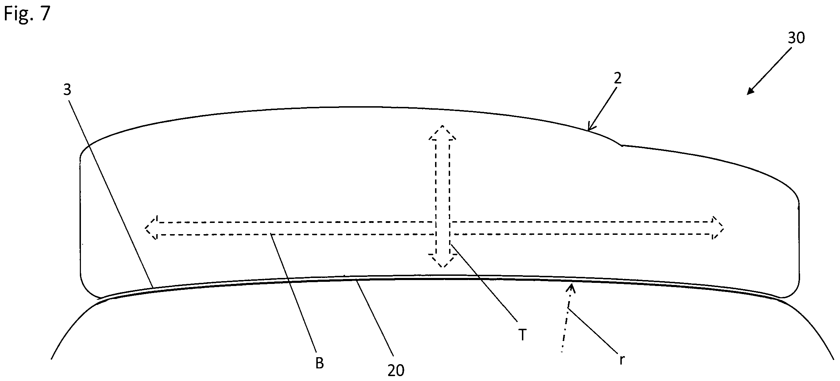

In den Figuren 6 und 7 ist ein Gehäuse 2 der Gasaustauschvorrichtung 30 und der erste Halteriemen 20 der Tragevorrichtung 1 des Tragesystems wiedergegeben. Das Tragesystem stellt dabei eine tragbares, medizinisches Gasaustauschersystem dar, das die vorbeschriebene Tragevorrichtung 1 und die ebenfalls vorbeschriebene Gasaustauschervorrichtung 30 miteinander vereint.

Wie den Fig. 6 und 7 zu entnehmen ist, erstreckt sich das Gehäuse 2 mit einer durchschnittliche Breite B, einer senkrecht zu dieser Breite B verlaufenden durchschnittliche Höhe H und einer senkrecht zur Breite B und zur Höhe H verlaufende durchschnittliche Tiefe T. Vorliegend wird von durchschnittlicher Breite, Höhe und Tiefe gesprochen, da das Gehäuse 2 eine "organische" Form aufweist, mit gerundeten Seiten und im Wesentlichen frei von Ecken ist. Die Tiefe T erstreckt sich dabei von einer Rückwand 3 des Gehäuses 2 weg. Die Tiefe T weist dabei eine maximale Größe von 20%, vorzugsweise 15%, der summarischen Gesamtlänge bzw. der Summe aus Breite B + Höhe H + Tiefe T auf. Die Gasaustauschvorrichtung weist dadurch eine flache Gestalt auf, die im Anwendungszustand nur einen sehr kleinen Hebel auf den Patienten ausübt, so dass die Gasaustauschvorrichtung über längere Zeit getragen werden kann, ohne den Patienten zu ermüden.

Wie insbesondere aus Fig. 7 ersichtlich ist, ist die dem ersten Halteriemen 20 zugewandte Rückwand 3 des Gehäuses 2 zum ersten Halteriemen 20 hin nach innen (konkav) gekrümmt, während der erste Halteriemen 20 zumindest im geschlossenen Zustand des Verschlusses 24 nach radial außen (konvex) gekrümmt ist, wobei der Krümmungsradius r bereichsweise unterschiedlich ist, wie insbesondere aus Fig. 1 bis 5 ersichtlich ist.

Die Krümmung der Rückwand 3 korrespondiert im Wesentlichen mit der Krümmung des ersten Halteriemens 20, so dass die Rückwand 3 des Gehäuses 2 im Wesentlichen bündig an dem nach radial außen gekrümmten ersten Halteriemen 20 anliegt, wodurch die Gasaustauschvorrichtung 30 des Tragesystems bzw. des tragbaren, medizinischen Gasaustauschersystems nahe am Körper eines Patienten zu liegen kommt, was den Tragekomfort erhöht und die Belastung des Patienten auf Grund eines geringen Hebels minimiert.

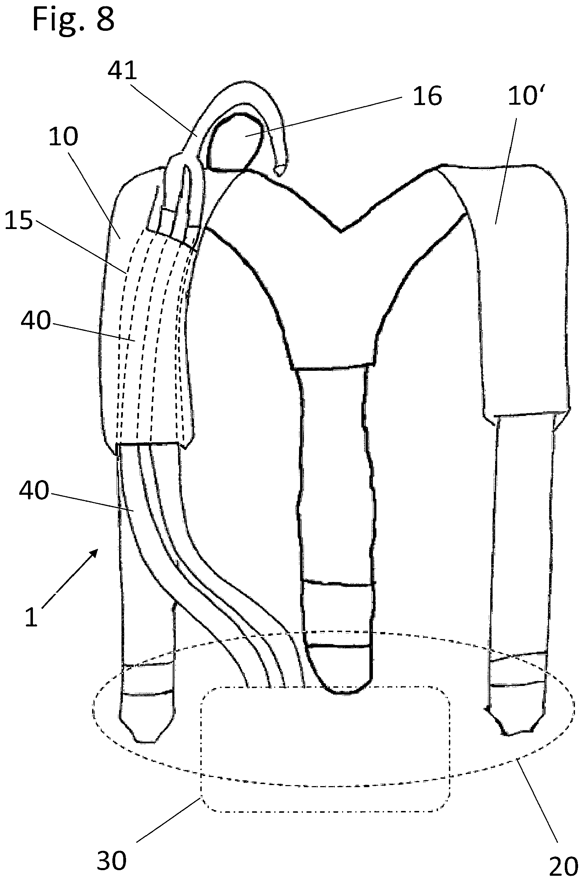

In Fig. 8 ist eine weitere Ausführungsform einer erfindungsgemäßen Tragevorrichtung 1 dargestellt, bei der der erste Halteriemen 20 lediglich andeutungsweise dargestellt ist, ebenso wie die Gasaustauschvorrichtung 30. Die in Fig. 8 dargestellte Ausführungsform einer Tragevorrichtung 1 für eine Gasaustauschvorrichtung 30 unterscheidet sich von der in Fig. 1 dargestellten Ausführungsform insbesondere dadurch, dass an einem zweiten Halteriemen 10, welcher wahlweise ein linker oder ein rechter Halteriemen 10 sein kann, ein Führungstunnel 15 für Schläuche 40, insbesondere blutführende Schläuche 40, welche vom und zum Gasaustauscher 30 verlaufen und im Einsatzfall der Tragevorrichtung 1 und der Gasausstauschvorrichtung 30 über eine Doppelkanüle 41 mit dem Blutkreislauf eines Patienten verbindbar sind. Im Schulterbereich des Halteriemens 10, welcher den Führungstunnel 15 aufweist, ist ferner noch ein Stütz- bzw. Führungselement 16 vorgesehen, über welches ein Abschnitt der Doppelkanüle 41 geführt ist, um in einer definierten Position relativ zu einem Patienten zu liegen zu kommen. In der vorliegenden Ausführungsform sind die beiden Halteriemen 10, 10' im rückwärtigen Abschnitt miteinander verbunden, so dass sich eine Y-förmige Geometrie ergibt.

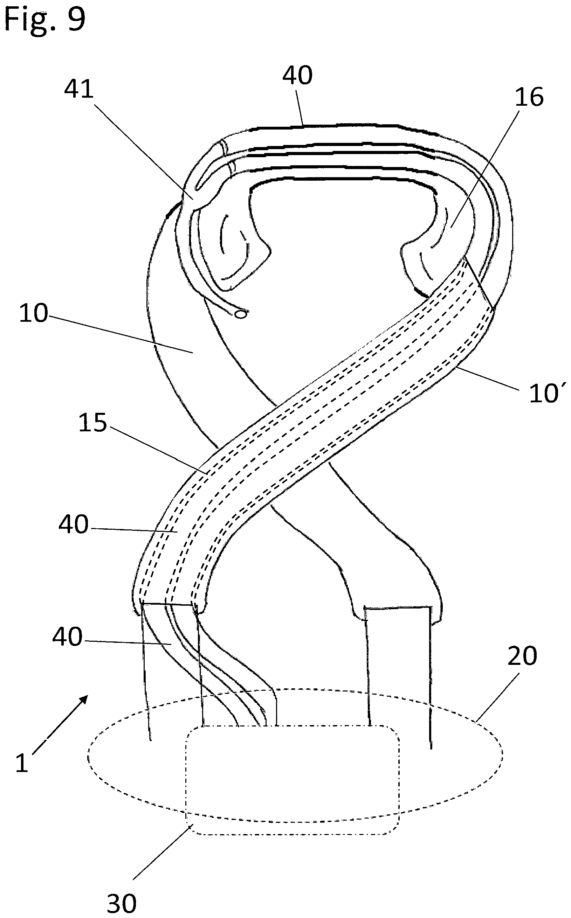

In Fig. 9 ist eine weitere alternative Ausführungsform einer erfindungsgemäßen Tragevorrichtung 1 dargestellt, die sich dadurch von der vorhergehenden unterscheidet, dass die Tragevorrichtung 1 keinen rückwärtigen Abschnitt aufweist, und dass die beiden Halteriemen 10, 10' hinter einem Kragenabschnitt 16 der Tragevorrichtung 1 miteinander verbunden sind, wo sie in einer Schlaufe um den Kragenabschnitt 16 herumgeführt sind. In einem der Halteriemen 10' ist wiederum ein Führungstunnel 15 zur Führung von, insbesondere blutführenden, Schläuchen 40 von der Gasaustauschvorrichtung 30 zum Patienten hin vorhanden. Die blutführenden Schläuche 40 enden an dem Kragenabschnitt 16 wiederum in einer Doppelkanüle 41, die so an dem Kragenabschnitt 16 geführt ist, dass diese wieder für einen optimalen Zugang zum Blutkreislauf eines Patienten, welcher die Tragevorrichtung und die Gasaustauschvorrichtung trägt, positioniert ist.

Es versteht sich, dass zusätzlich zu den hier beispielhaft dargelegten Ausführungsformen weitere alternative Ausführungsformen im Rahmen der vorliegenden Erfindung denkbar sind. Es versteht sich ferner, dass die vorliegende Erfindung für eine Reihe von Materialien, wie bspw. natürliche oder synthetische Stoffe, elastische oder nichtelastische Stoffe atmungsaktive, reißfeste, wasserabweisende, sterile bzw. sterilisierbare und andere Stoffe gleichermaßen anwendbar ist. Zudem können die Stoffe miteinander verwebbar, vernähbar, verschmelzbar oder auf andere Weise miteinander verbindbar sein.

Im Übrigen können an der Tragevorrichtung, insbesondere an Stellen, an denen ein intensiver Kontakt mit dem Körper eines Patienten, wie bspw. an den Schultern oder der Hüfte eines Patienten, vorherrscht, Polster, Polsterungen oder zusätzliche Stoffe oder Mittel vorgesehen sein, ohne sich von dem Erfindungsgegenstand zu entfernen.

In einigen Ausführungsformen, in denen die Gasaustauschvorrichtung einen Umgebungsluftansaugabschnitt aufweist, kann das Ansaugen der Umgebungsluft unmittelbar an der Gasaustauschvorrichtung erfolgen. Alternativ kann der Umgebungsluftansaugabschnitt einen Ansaugschlauch aufweisen, der entlang der Tragevorrichtung geführt ist, beispielsweise ähnlich zu einem Schlauch des extrakorporalen Blutkreislaufs oder einem Kabel der Steuereinheit und an einer vorbestimmten Stelle der Tragevorrichtung Luft ansaugen. Insbesondere kann so eine Luft in einem Kopfbereich eines Patienten angesaugt werden, wo sich gegebenenfalls weniger Schwebeteilchen in der Luft befinden. Zudem kann auf diese Weise ein Bedecken des Umgebungsluftansaugabschnitts, beispielsweise durch Kleidung vor der Gasaustauschvorrichtung, und eine damit verbundene Reduktion der zuführbaren Luft, vermieden werden.

## Patentansprüche

1. Tragevorrichtung (1) für eine tragbare Gasaustauschvorrichtung, aufweisend
- wenigstens einen ersten Halteriemen (20), der dazu ausgelegt ist, um einen Torso eines Patienten geführt zu werden und auf diese Weise eine Abstützung der Tragevorrichtung (1) an dem Patienten ermöglicht,
- wenigstens einen zweiten Halteriemen (10), der dazu ausgelegt ist, um wenigstens eine erste Schulter eines Patienten geführt zu werden, sowie
- wenigstens ein Befestigungsmittel (26, 27), das zur Befestigung einer tragbaren Gasaustauschvorrichtung ausgelegt ist,
wobei der zweite Halteriemen (10) mit dem ersten Halteriemen (20) verbunden ist oder, zumindest abschnittsweise, um den ersten Halteriemen (20) geführt wird.

2. Tragevorrichtung (1) nach Anspruch 1, wobei der zweite Halteriemen (10) dazu ausgelegt ist, eine Gewichtskraft der Tragevorrichtung (1) und daran befestigter Komponenten abzuleiten.

3. Tragevorrichtung (1) nach einem der Ansprüche 1 oder 2, wobei der wenigstens erste (20) und/oder zweite Halteriemen (10) ein Mittel (11a, 11b, 24) zur Anpassung der Länge des ersten (20) und/oder zweiten Haltriemens (10) aufweist.

4. Tragevorrichtung (1) nach einem der vorhergehenden Ansprüche, wobei die Tragevorrichtung (1) einen dritten Halteriemen (10') aufweist, der vorzugsweise symmetrisch zu dem zweiten Halteriemen (10) ausgebildet ist, und dazu ausgelegt ist, um eine zweite Schulter eines Patienten geführt zu werden.

5. Tragevorrichtung (1) nach einem der vorhergehenden Ansprüche, wobei die Tragevorrichtung (1) wenigstens einen Verschluss (12, 14a, 14b, 24) zum Öffnen und Schließen wenigstens eines der Halteriemen (10, 10', 20) aufweist, wobei optional wenigstens ein Verschluss (12, 14a, 14b, 24) einen Haftverschluss und/oder einen Klemmverschluss und/oder einen Eingriffsverschluss umfasst.

6. Tragevorrichtung (1) nach Anspruch 5, wobei wenigstens ein Verschluss (12, 14a, 14b, 24) in einem geschlossenem Zustand den zweiten (10) bzw. dritten Halteriemen (10') mit dem ersten Halteriemen (20) verbindet.

7. Tragevorrichtung (1) nach einem der Ansprüche 1 bis 5, wobei wenigstens zwei der Halteriemen (10, 10', 20) einstückig miteinander ausgebildet sind.

8. Tragevorrichtung (1) nach einem der vorhergehenden Ansprüche, wobei die Tragevorrichtung (1) des Weiteren eine Kabelaufnahme (22), insbesondere an dem ersten Halteriemen (20), aufweist, die ausgebildet ist, wenigstens ein Kabel (32), insbesondere an dem ersten Halteriemen (20), zu halten und/oder zu sichern.

9. Tragesystem aufweisend eine Tragevorrichtung (1) gemäß einem der vorhergehenden Ansprüche 1 bis 8 sowie eine tragbare Gasaustauschvorrichtung (30), wobei die Gasaustauschvorrichtung (30) ein Befestigungsmittel aufweist, das komplementär zu einem Befestigungsmittel (26) der Tragevorrichtung (1) ausgebildet ist, derart, dass die Gasaustauschvorrichtung (30) an der Tragevorrichtung (1), vorzugsweise reversibel, befestigbar ist.

10. Tragesystem nach Anspruch 9, wobei die Gasaustauschvorrichtung (30) eine Steuereinheit (34) aufweist, und wobei die Steuereinheit (34) ein Befestigungsmittel (35) aufweist, das zur Befestigung an der Tragevorrichtung (1) und/oder an der Gasaustauschvorrichtung (30) ausgebildet ist.

11. Tragesystem nach Anspruch 10, wobei die Gasaustauschvorrichtung (30) ein Kabel (32) zur Verbindung mit der Steuereinheit (34) aufweist, wobei das Kabel (32) in einer an der Tragevorrichtung (1) ausgebildeten Kabelaufnahme (22) aufnehmbar ist, oder
wobei die Gasaustauschvorrichtung (30) und/oder die Steuereinheit (34) eine Sende- und/oder Empfangseinheit zur kabellosen Verbindung der Gasaustauschvorrichtung (30) mit der Steuereinheit (34) aufweist.

12. Tragesystem nach einem der Ansprüche 10 oder 11, wobei das Tragesystem, vorzugsweise die Gasaustauschvorrichtung des Tragesystems, einen Umgebungsluftansaugabschnitt aufweist.

13. Tragesystem nach Anspruch 9, wobei die Gasaustauschvorrichtung (30) ein Gehäuse (2) aufweist, das eine dem ersten Halteriemen (20) zugewandte Rückwand (3) aufweist, die zum ersten Halteriemen (20) hin nach innen gekrümmt ist.

14. Tragesystem nach Anspruch 13, wobei die Krümmung der Rückwand (3) mit der Krümmung des ersten Halteriemens (20) im Wesentlichen korrespondiert, so dass die Rückwand (3) des Gehäuses (2) im Wesentlichen bündig an dem nach radial außen gekrümmten ersten Halteriemen (20) anliegt.

15. Tragesystem nach Anspruch 13 oder 14, wobei das Gehäuse (2) eine durchschnittliche Breite (B), eine senkrecht zu dieser Breite (B) verlaufende durchschnittliche Höhe (H) und eine senkrecht zur Breite (B) und zur Höhe (H) verlaufende durchschnittliche Tiefe (T) aufweist, wobei sich die Tiefe (T) von der Rückwand (3) weg erstreckt und wobei die Tiefe (T) eine maximale Größe von 20%, vorzugsweise 15% der summarischen Gesamtlänge aus Breite (B) + Höhe (H) + Tiefe (T) aufweist.

## Claims

1. Carrying device (1) for a portable gas exchange device, having:
- at least one first carrying strap (20) which is designed to go around a patient's torso and thereby allow the carrying device (1) to be supported on the patient,
- at least one second carrying strap (10) that is designed to go over at least a first shoulder of a patient, and
- at least one fastening means (26, 27) that is designed to fasten a portable gas exchange device,
wherein the second carrying strap (10) is connected with the first carrying strap (20) or, at least sectionwise, goes around the first carrying strap (20).

2. Carrying device (1) according to claim 1, wherein the second carrying strap (10) is designed to deflect weight of a carrying device (1) and components attached thereto.

3. Carrying device (1) according to any one of claims 1 or 2, wherein the at least one first (20) and/or second (10) carrying strap has a means (11a, 11b, 24) for adapting the length of the first (20) and/or second carrying strap (10).

4. Carrying device (1) according to any one of the preceding claims, wherein the carrying device (1) has a third carrying strap (10') that is preferably designed to be symmetrical to the second carrying strap (10), and is configured to go around a second shoulder of a patient.

5. Carrying device (1) according to any one of the preceding claims, wherein the carrying device (1) has at least one closing means (12, 14a, 14b, 24) for opening and closing at least one of the carrying straps (10, 10', 20), wherein optionally at least one closing means (12, 14a, 14b, 24) comprises an adherence fastener, and/or a clamping fastener, and/or an engaging fastener.

6. Carrying device (1) according to claim 5, wherein at least one closing means (12, 14a, 14b, 24) connects the second (10) and/or third carrying strap (10') to the first carrying strap (20) when in a closed state.

7. Carrying device (1) according to one of claims 1 through 5, wherein at least two of the carrying straps (10, 10', 20) are designed together as a single piece.

8. Carrying device (1) according to any one of the preceding claims, wherein the carrying device (1) further has, in particular on the first carrying strap (20), a cable holder (22) that is designed to hold and/or secure at least one a cable (32), in particular on the first carrying strap (20).

9. Carrying system having a carrying device (1) according to any one of the preceding claims 1 through 8, as well as a portable gas exchange device (30), wherein the gas exchange device (30) has a fastening means that is designed to be complementary to a fastening means (26) of the carrying device (1), such that the gas exchange device (30) can be fastened, preferably reversibly, to the carrying device (1).

10. Carrying system according to claim 9, wherein the gas exchange device (30) has a control unit (34), and wherein the control unit (34) has a fastening means (35) that is designed to fasten to the carrying device (1) and/or to the gas exchange device (30).

11. Carrying system according to claim 10, wherein the gas exchange device (30) has a cable (32) for connecting to the control unit (34), wherein the cable (32) can be held in a cable holder (22) formed on the carrying device (1) or
wherein the gas exchange device (30) and/or the control unit (34) has a transmitting and/or receiving unit for wirelessly connecting the gas exchange device (30) to the control unit (34).

12. Carrying system according to any one of claims 10 or 11, wherein the carrying system, preferably the gas exchange device of the carrying system, has an ambient air suction section.

13. Carrying system according to claim 9, wherein the gas exchange device (30) has a housing (2) that has a rear wall (3) that faces the first carrying strap (20) and is curved inward toward the first carrying strap (20).

14. Carrying system according to claim 13, wherein the curvature of the rear wall (3) essentially corresponds to the curvature of the first carrying strap (20), such that the rear wall (3) of the housing (2) essentially abuts flushly against the first carrying strap (20) curved radially to the outside.

15. Carrying system according to claim 13 or 14, wherein the housing (2) has an average width (B), an average height (H) running perpendicular to this width (B), and an average depth (T) running perpendicular to the width (B) and height (H), wherein the depth (T) extends away from the rear wall (3), and wherein the depth (T) has a maximum size of 20%, preferably 15%, of the summary overall length of width (B) + height (H) + depth (T).

## Revendications

1. Dispositif de support (1) pour un dispositif d'échange gazeux portable, comprenant :
- au moins une première lanière de maintien (20) qui est configurée pour être passée autour du torse d'un patient, et qui permet de cette manière un appui du dispositif de support (1) contre le patient,
- au moins une deuxième lanière de maintien (10) qui est configurée pour être passée au moins autour d'une première épaule d'un patient, ainsi que
- au moins un moyen de fixation (26, 27) qui est configuré pour la fixation d'un dispositif d'échange gazeux portable,
dans lequel la deuxième lanière de maintien (10) est reliée à la première lanière de maintien (20), ou, au moins en partie, est passée autour de la première lanière de maintien (20).

2. Dispositif de support (1) selon la revendication 1, dans lequel la deuxième lanière de maintien (10) est configurée pour dévier une force due au poids du dispositif de support (1) et des composants qui lui sont fixés.

3. Dispositif de support (1) selon l'une des revendications 1 ou 2, dans lequel au moins la première lanière de maintien (20) et/ou la deuxième lanière de maintien (10) comporte(nt) un moyen (11a, 11b, 24) pour ajuster la longueur de la première lanière de maintien (20) et/ou la deuxième lanière de maintien (10).

4. Dispositif de support (1) selon l'une des revendications précédentes, dans lequel le dispositif de support (1) comporte une troisième lanière de maintien (10') qui est réalisée de préférence symétriquement à la deuxième lanière de maintien (10) et qui est configurée pour être passée autour de la deuxième épaule d'un patient.

5. Dispositif de support (1) selon l'une des revendications précédentes, dans lequel le dispositif de support (1) comporte au moins un dispositif de fermeture (12, 14a, 14b, 24) pour ouvrir et fermer au moins une des lanières de maintien (10, 10', 20), au moins un dispositif de fermeture (12, 14a, 14b, 24) comprenant, en option, une fermeture adhésive et/ou une fermeture par serrage et/ou une fermeture à contact.

6. Dispositif de support (1) selon la revendication 5, dans lequel au moins un dispositif de fermeture (12, 14a, 14b, 24) relie, à l'état de fermeture, la deuxième lanière de maintien (10) ou la troisième lanière de maintien (10') à la première lanière de maintien (20).

7. Dispositif de support (1) selon l'une des revendications 1 à 5, dans lequel au moins deux des lanières de maintien (10, 10', 20) sont réalisées ensemble d'une seule pièce.

8. Dispositif de support (1) selon l'une des revendications précédentes, dans lequel le dispositif de support (1) comporte, en outre, un logement de câble (22), en particulier au niveau de la première lanière de maintien (20), qui est réalisée afin au moins de maintenir et/ou de sécuriser un câble (32), en particulier au niveau de la première lanière de maintien (20).

9. Système de support comportant un dispositif de support (1) selon l'une des revendications précédentes 1 à 8, ainsi qu'un dispositif d'échange gazeux (30) portable, dans lequel le dispositif d'échange gazeux (30) comporte un moyen de fixation, qui est réalisé en complément à un moyen de fixation (26) du dispositif de fixation (1), de telle sorte que le dispositif d'échange gazeux (30) peut être fixé, de préférence de manière réversible, au niveau du dispositif de support (1).

10. Système de support selon la revendication 9, dans lequel le dispositif d'échange gazeux (30) comporte une unité de commande (34) et dans lequel l'unité de commande (34) comporte un moyen de fixation (35) qui est réalisé pour la fixation au niveau du dispositif de support (1) et/ou au niveau du dispositif d'échange gazeux (30).

11. Système de support selon la revendication 10, dans lequel le dispositif d'échange gazeux (30) comporte un câble (32) pour liaison à l'unité de commande (34), le câble (32) pouvant être logé dans un logement de câble (22) réalisé au niveau du dispositif de support (1), ou dans lequel le dispositif d'échange gazeux (30) et/ou l'unité de commande (34) comporte une unité de transmission et/ou une unité de réception pour la liaison sans câble du dispositif d'échange gazeux (30) avec l'unité de commande (34).

12. Système de support selon l'une des revendications 10 ou 11, dans lequel le système de support, de préférence le dispositif d'échange gazeux du système de support, comporte une section d'admission d'air ambiant.

13. Système de support selon la revendication 9, dans lequel le dispositif d'échange gazeux (30) comporte un boitier (2) qui comporte une paroi arrière (3) orientée vers la première lanière de maintien (20) qui est courbée vers l'intérieur en direction de la première lanière de maintien (20).

14. Système de support selon la revendication 13, dans lequel la courbure de la paroi arrière (3) correspond essentiellement à la courbure de la première lanière de maintien (20), de sorte que la paroi arrière (3) du boitier (2) est essentiellement affleurante au niveau de la première lanière de maintien (20) courbée radialement vers l'extérieur.

15. Système de support selon la revendication 13 ou 14, dans lequel le boitier (2) comporte une largeur (B) moyenne, une hauteur (H) moyenne, perpendiculaire à cette largeur (B), et une profondeur (T) moyenne, perpendiculaire à la largeur (B) et à la hauteur (H), la profondeur (T) s'étendant en s'éloignant de la paroi arrière (3) et la profondeur (T) comportant une dimension maximale de 20 %, de préférence 15 %, de la longueur totale cumulée à partir de la largeur (B) + la hauteur (H) + la profondeur (T).
